# EUROPEAN PATENT SPECIFICATION

(11) **EP 4 277 520 B1**
(45) Date of publication and mention of the grant of the patent: **20.05.2026**
(21) Application number: 22740201.3
(22) Date of filing: 16.01.2022
(51) Int. Cl.: A61B 5/00, A61B 5/08, A61B 5/087

(54) **MONITORING AIRFLOW WITH B-MODE ULTRASOUND**
ÜBERWACHUNG DES LUFTSTROMS MIT B-MODUS-ULTRASCHALL
SURVEILLANCE DE CIRCULATION D'AIR AVEC DES ULTRASONS EN MODE B

(30) Priority: 15.01.2021 US 202163137959 P
(43) Date of publication of application: 22.11.2023
(73) Proprietor: Wisconsin Alumni Research Foundation, Madison, WI 53726 (US)
(72) Inventor: BILEN-ROSAS, Guelay, Middleton, WI 53562 (US); ROSAS, Humberto, Gerardo, Middleton, WI 53562 (US); ONG, Irene, May, Lin, Fitchburg, WI 53711 (US)
(74) Representative: Samson & Partner Patentanwälte mbB
(86) International application number: PCT/US2022/012650
(87) International publication number: WO 2022/155559

(56) References cited:
- US-A1- 2013 289 401
- US-A1- 2015 238 169
- US-A1- 2018 256 075
- US-A1- 2019 167 227
- LIU MENGHAN; HUANG MING-CHUN: "Asthma Pattern Identification via Continuous Diaphragm Motion Monitoring", IEEE TRANSACTIONS ON MULTI-SCALE COMPUTING SYSTEMS, vol. 1, no. 2, 1 June 2015 (2015-06-01), pages 76 - 84, XP011594454, DOI: 10.1109/TMSCS.2015.2496214
- HARPUT SEVAN; CHRISTENSEN-JEFFRIES KIRSTEN; LI YUANWEI; BROWN JEMMA; ECKERSLEY ROBERT J.; DUNSBY CHRIS; TANG MENG-XING: "Two stage sub-wavelength motion correction in human microvasculature for CEUS imaging", 2017 IEEE INTERNATIONAL ULTRASONICS SYMPOSIUM (IUS), 6 September 2017 (2017-09-06), pages 1 - 4, XP033245657, DOI: 10.1109/ULTSYM.2017.8092538
- UEKI J, DE BRUIN P F, PRIDE N B: "In vivo assessment of diaphragm contraction by ultrasound in normal subjects", THORAX, vol. 50, no. 11, 1 November 1995 (1995-11-01), GB , pages 1157 - 1161, XP055958942, ISSN: 0040-6376, DOI: 10.1136/thx.50.11.1157

## Description

### BACKGROUND

In the past few decades, advances in monitoring technology have contributed to a heightened sense of safety during medical procedures. Despite technological advancements and improved anesthetic agents leading to an increased safety profile of anesthesia and sedation, morbidity and mortality rates remain high in spontaneously breathing patients. One of the primary reasons morbidity and mortality rates remain high is the delayed detection of early respiratory compromise, which impedes the timely implementation of rescue measures. The end results of respiratory compromise is insufficient oxygen to the brain and heart, leading to grave consequences including permanent neurological and cardiac damage, or even death. Currently available monitoring devices and techniques continue to be ineffective at measuring subtle airflow changes in the early stages of respiratory compromise.

Respiratory compromise may include one or more of the following scenarios: alterations in respiratory rate, decreased effort, obstruction in the upper airway anatomy (e.g., tongue obstruction, tissue obstruction, vocal cord spasms), alterations in lower airway structures (e.g., bronchospasm, pneumonia), and apnea (central or obstructive). Patients under sedation can experience decreased respiratory effort and varying degrees of tissue laxity, which can lead to airway obstruction and apnea, both of which are difficult to assess.

Because of the lack of reliable respiratory monitoring, early recognition of respiratory compromise relies heavily on clinical expertise. Accurately monitoring for respiratory compromise is especially difficult in pediatric patients, whose anatomy makes maintaining airway and respiratory homeostasis challenging during inhalation induction for anesthesia or sedation. The head and neck anatomy of pediatric patients, as well as their unique respiratory physiology, predisposes this patient population to a higher incidence of airway obstruction and rapid rate of desaturation. For example, pediatric patients experience higher rates of tongue obstruction during sedation and anesthesia due to their larger tongue size in relation to the oropharynx than adult patients. Additionally, at baseline, pediatric patients have two- to three-fold higher oxygen consumption; decreased functional capacity leading to diminished oxygen reserves once apneic; decreased type-1 muscle fibers resulting in faster respiratory fatigue during times of labor; and a closing capacity that is at the level of tidal volume breathing.

Any loss in the degree of respiratory effort brought about by sedation/anesthesia or respiratory illness will tip the balance from staying stable and ventilating appropriately to quick ventilatory compromise, respiratory deterioration with decrease in airflow, and desaturation (i.e., type-2 respiratory failure). Unrecognized and delayed respiratory support requiring skilled airway maneuvers (e.g., hand-bag-ventilation) will lead to severe desaturation followed by bradycardia, which can be fatal for the patient.

As mentioned above, the currently available methods for monitoring respiratory compromise are ineffective at directly measuring airflow changes in non-intubated patients, thereby rendering the methods unreliable for accurately and timely detecting early respiratory compromise.

One of the most common methods currently employed for monitoring airflow is measuring end-tidal carbon dioxide ("CO2"). However, measuring end-tidal CO2 has inherent limitations because it is an indirect measurement of alterations in airflow and is inaccurate and non-quantitative in non-intubated patients due to the lack of a closed circuit. As a consequence, the measured data are difficult for the practitioner to interpret in spontaneously breathing patients, which often leads to delays in the treatment of early airway compromise.

Another common method for monitoring respiratory compromise is pulse oximetry, which indirectly measures a patient's oxygen saturation, and which is a standard American Society of Anesthesiologists ("ASA") monitor in operating rooms and most office-based sedation cases. However, pulse oximetry does not directly monitor respiration and hence does not monitor ventilation. For example, an obstructed airway will decrease oxygen flow and hence oxygen supply to the body, leading to desaturation (i.e., a drop in oxygen). The limitation of pulse oximetry is a delayed response to desaturation, resulting in a time lag in the detection of hypoxic events, particularly in the presence of supplemental oxygen.

Electrocardiography ("ECG") monitoring, a non-respiratory monitor, can also be used, but only shows changes in heart rate (e.g., bradycardia) once arterial oxygen desaturation has exceeded a critical point and cardiac cells have become damaged from a lack of oxygen. Thus, like end-tidal CO2 and pulse oximetry, ECG only indirectly measures respiratory compromise by displaying changes in heart function (e.g., drop in blood pressure and heart rate) due to decreased oxygen supply to the heart as the consequence of airflow deterioration. Moreover, ECG-monitoring does not provide real-time measurements necessary to timely identify early airway compromise.

Thoracic impedance monitoring can also be used for post-operative respiratory rate assessment. This measurement technique, however, is very susceptible to erroneous readings secondary to motion artifact. For instance, this methodology will continue to record a respiratory rate despite the patient breathing against a closed glottis, a situation in which airflow has ceased partially or completely.

Presently, the success of early detection of respiratory compromise relies heavily on physician or other healthcare provider expertise, which introduces significant inter-provider variability. A non-invasive method of quantifying small changes in airflow patterns would allow physicians with various degrees of experience to detect early respiratory compromise, specifically in the outpatient setting where sedation is delivered by non-anesthesiologists; in the ICU where pain management, particularly with opioids, can lead to over-sedation; in the post-anesthesia recovery unit where patients are still awakening from anesthesia; in the emergency room where patients are presenting with respiratory issues due to trauma, reactive airway exacerbations, or infection; and in the operating room for inhalation and intravenous induction.

US 2015/238169 A1 discloses a computerized method for analyzing ultrasound signals, the steps of the method comprising:
a. receiving B-mode signals with a computer system, wherein the B-mode signals were previously acquired using an ultrasound system to acquire the B-mode signals from tissue in a region-of-interest;
b. computing B-mode motion signal data from the B-mode signals using the computer system, wherein the B-mode motion signal data indicate motion that occurred in the region-of-interest while the B-mode signals were acquired; and
c. estimating from the B-mode motion signal data, respiratory parameter data.

Thus, there remains a need for a non-invasive system and method for directly and timely monitoring for respiratory compromise and airflow changes in patients. Such a system and method would be advantageous not only for clinical and outpatient settings, but also for research and teaching applications. Such a tool would prompt timely airway rescue and reduce the morbidity and mortality rates associated with undetected respiratory compromise.

### SUMMARY OF THE DISCLOSURE

The present disclosure addresses the aforementioned drawbacks by providing a method according to claim 1.

The foregoing and other aspects and advantages of the present disclosure will appear from the following description. In the description, reference is made to the accompanying drawings that form a part hereof, and in which there is shown by way of illustration a preferred embodiment. This embodiment does not necessarily represent the full scope of the invention, however, and reference is therefore made to the claims and herein for interpreting the scope of the invention.

### BRIEF DESCRIPTION OF THE DRAWINGS

FIG. 1 is a flowchart setting forth the steps of an example method for monitoring and/or quantifying parameters of a subject's respiration based on B-mode ultrasound imaging.
FIGS. 2A and 2B show example B-mode images and corresponding B-mode displacement estimated from those B-mode images together with volumetric flow rate as a function of time.
FIG. 3 shows a scatter plot of example B-mode displacement as a function of volumetric flow.
FIG. 4 shows the detection of a swallow event from example B-mode motion signal data.
FIG. 5 is a flowchart setting forth the steps of an example method for estimating respiratory parameter data from B-mode ultrasound signals using a trained machine learning algorithm.
FIG. 6 is a flowchart setting forth the steps of an example method for training a machine learning algorithm to estimate respiratory parameter data from B-mode ultrasound signals.
FIG. 7 shows an example of different B-motion window sizes that can be used when assembling training data.
FIG. 8 is a block diagram of an example ultrasound system that can implement the methods described in the present disclosure.
FIG. 9 is a block diagram of an example airway monitor system according to some embodiments described in the present disclosure.
FIG. 10 is a block diagram of example components that can implement the airway monitor system of FIG. 9.

### DETAILED DESCRIPTION

Described here are systems and methods for monitoring airflow changes in a subject's airway. This monitoring may be performed before, during, or following a procedure, or may be used as a general patient monitoring tool. For example, the systems and methods of the present disclosure can be used to monitor signs of airway obstruction or respiratory compromise and/or failure during timeframes where patients have ongoing or residual sedation or anesthetics in their system. Additionally or alternatively, the systems and methods described in the present disclosure can be used to monitor a subject's airway to detect conditions such as sleep apnea and/or chronic obstructive pulmonary disease ("COPD"). In still other applications, the systems and methods described in the present disclosure can be used to monitor post-operative respiratory compromise/failure in recovery rooms after surgery and/or on the floor, or alerting doctors and/or patients about their worsening respiratory conditions such as asthma and/or emphysema.

The systems and methods can also be implemented for quantitatively measuring airflow and related parameters. As one example, the systems and methods of the present disclosure can be used to quantitatively measure respiratory rate in real-time. As other examples, the systems and methods of the present disclosure can be used to quantitatively measure tidal volume, minute ventilation, the inspiratory and expiratory phase of breath, flow properties such as velocity and Reynolds number, and so on. In addition, the systems and methods described in the present disclosure provide an improved accuracy because they are capable of detecting actual breaths as opposed to attempted breaths, such as in upper airway obstruction when the chest wall continues to make efforts against a closed glottis.

Moreover, the systems and methods described in the present disclosure are capable of detecting changes in airflow patterns, such as turbulent flow and laminar flow. In some embodiments, in addition to B-mode ultrasound data, pulsed wave Doppler data may be obtained, such that sound pressure gradient changes at an air-media interface may be additionally detected and monitored, such as an air-soft tissue interface. In this way, the propagation of sound waves in air can also be detected and tracked.

Presently available monitors display information for respiratory ventilation via indirect measurements (e.g., via measuring changes in CO₂ and O₂). These currently available monitors do not provide direct quantitative measurements of airflow and perform inaccurately in spontaneously breathing patients. Moreover the data provided by these currently available monitors is significantly delayed, and therefore these monitors often cannot timely indicate early airway compromise and/or failure.

The systems and methods described here utilize B-mode ultrasound to measure changes in the airway (e.g., tracheal wall, cricothyroid ligament) during inspiration and expiration. With B-mode ultrasound data, the direction and total displacement of the imaged tissues can be measured and correlated with respiratory phase information, airflow, and related quantitative parameters. By comparing properties of these B-mode data, and displacement data derived therefrom, to a baseline signal, which may include normative data, respiratory compromise and/or failure (e.g., airflow obstruction) can be identified, in addition to respiratory phase. Further, properties and parameters of airflow and respiration can be computed or otherwise estimated. Additionally or alternatively, the B-mode data and/or displacement data can be input to a trained machine learning algorithm, such as a trained neural network, generating output as qualitative or quantitative data indicative of respiratory compromise and/or failure (e.g., airflow obstruction), which in some instances may include values or maps of properties and/or parameters of airflow and respiration.

As an example, respiratory compromise and/or failure can be identified as a percent change (e.g., decrease) in imaged motion depicted in B-mode images relative to the baseline signal, relative to the expected motion based on normative data, or as output generated by inputting the B-mode images and/or related displacement data to a trained machine learning algorithm (e.g., a trained neural network). The systems and methods described in the present disclosure thus provide a real-time measurement of airflow that can be monitored and directly related to detect respiratory compromise and/or failure, such as airway obstruction.

Monitoring airflow using B-mode data has several advantages. As one example, the B-mode data enables better prediction of respiratory rate, which is an important metric for monitoring a patient's ventilation and/or breathing status. This suite of technologies has the potential to provide medical professionals advanced notice of patient breathing status, which could minimize complications resulting from prolonged periods of oxygen deprivation. In particular, this system could help healthcare providers recognize respiratory depression and airway obstruction earlier so that they could initiate corrective and resuscitative measures.

During anesthesia, especially in pediatric anesthesia, the systems and methods described in the present disclosure can provide an accurate monitor for airway obstruction and early respiratory comprise, and/or failure leading to more timely intervention and reduced patient morbidity and mortality. The systems and methods described in the present disclosure can also provide an efficient teaching tool in the training of residents and fellows. For example, the measured values from this non-invasive monitor can be more tangibly correlated with clinical changes of the patient, which currently is not possible. Learning these clinical skills for the pediatric population otherwise requires many years of pediatric anesthesia practice. A teaching tool that can expedite this learning process would be advantageous.

The ultrasound-based monitor described in the present disclosure provides a method for detecting alterations in airflow using B-mode data within the subject's airway (e.g., along the tracheal wall, cricothyroid ligament). The noninvasiveness and continuous and instant collection of data makes this tool advantageous for collecting and displaying information about the changing dynamics of airflow in real-time. The systems and methods described in the present disclosure can therefore improve clinical judgment, practice, and teaching.

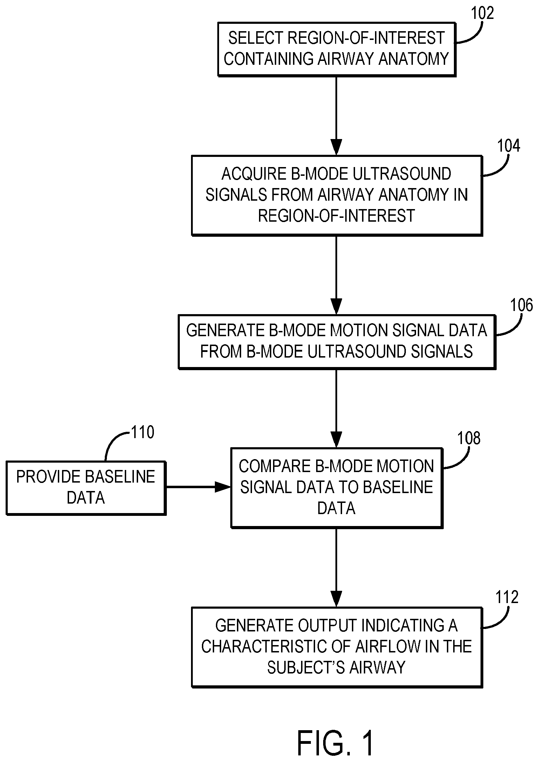

Referring now to FIG. 1, a flowchart is illustrated as setting forth the steps of an example method for monitoring a subject for respiratory compromise and/or failure using ultrasound. The method includes selecting a region-of-interest ("ROI") containing an anatomical region in the subject's airway (e.g., the subject's trachea, cricothyroid ligament, other connective or cartilaginous tissue in the trachea, larynx or pharynx), as indicated at step 102.

For example, the ROI can be selected by operating the ultrasound system to acquire B-mode images of the subject and identifying an ROI in those B-mode images that includes the desired anatomical region (e.g., the subject's trachea, cricothyroid ligament). In some instances, the ROI can be selected manually. In some other instances, the ROI can be automatically or semi-automatically selected based on the nature of how the anatomical region is depicted in a B-mode image. For example, the tracheal wall and cricothyroid ligament are hyperechoic in B-mode images and automated or semiautomated selection or segmentation technique can be implemented based on this hyperechoic nature.

In still other instances, the ROI can be selected using a machine learning algorithm. For example, a suitable machine learning algorithm can be trained to detect ROIs such that when B-mode images or other ultrasound data are input to the trained machine learning algorithm, an ROI is generated or otherwise selected. As one example, the machine learning algorithm may include a convolutional neural network that has been trained to identify ROIs containing particular anatomical targets such as those discussed in the present disclosure. For instance, a convolutional neural network such as a Mask R-CNN can be used to automatically segment and identify the ROI.

Ultrasound signals are then acquired from the ROI using the ultrasound system, as indicated at step 104. As noted above, the ultrasound signals are preferably acquired as B-mode ultrasound signals. In some instances, additional ultrasound data may also be acquired and/or accessed from storage, such as from a memory or other suitable data storage device or medium. As one example, the additional ultrasound data may include Doppler ultrasound signals. Preferably, the ultrasound signals are acquired while the ultrasound transducer is oriented such that the anatomical region (e.g., the tracheal wall, cricothyroid ligament) is being imaged in the longitudinal plane. In other instances, the anatomical region can be imaged in other planes, such as the transverse plane or an oblique plane. The ultrasound signals are preferably continuously recorded while being analyzed to monitor for airway obstruction or respiratory compromise and/or failure.

As one example, B-mode ultrasound can be used to detect airflow changes across the anatomical region (e.g., trachea, larynx, pharynx) It is a discovery of the present disclosure that displacement along the anatomical region within the subject's airway (e.g., tracheal wall, cricothyroid ligament) measured using B-mode ultrasound correspond to airflow changes, air pressure changes, sound pressure changes, or combinations thereof, in spontaneously breathing and non-intubated patients. Thus, measured changes in the velocities along the anatomical region (e.g., tracheal wall, cricothyroid ligament) can be associated with airflow changes, including airway obstruction or respiratory compromise and/or failure.

In one non-limiting example, the anatomical region (e.g., tracheal wall, cricothyroid ligament) can be imaged in the longitudinal plane using high-resolution ultrasound transducer (e.g., a 10-15 MHz transducer). B-mode data obtained from the anatomical region in the subject's airway can be used to quantify tissue movement along the anatomical region during the different phases of ventilation.

In some examples, ultrasound signals are continuously recorded from the ROI while the subject is breathing under anesthesia, sedation, or both. Additionally or alternatively, ultrasound signals can be continuously recorded under normal breathing conditions in which anesthesia and/or sedation are not used. In these applications, the ultrasound signals can be monitored for worsening breathing issues due to underlying respiratory problems such as asthma, emphysema, pneumonia, and/or hypersensitivity pneumonitis from exposures to various inhalants (e.g., dust, bird seed, asbestos). Alternatively, the ultrasound signals can be monitored to help in the evaluation of sleep apnea or for other respiratory conditions.

As one non-limiting example, during breathing, changes in airflow in the airway will be recorded as changes in the measured ultrasound signals and/or imaged tissue motion, which can be compared to baseline data that were acquired before the subject was placed under anesthesia, sedation, or both. As mentioned above, in some instances the baseline signal data may include normative data. A suitable correlation or other algorithm can be used to identify critical changes in the subject's airflow, relative to the baseline data, in real-time, as described below. Additionally or alternatively, as described in more detail below, a trained machine learning algorithm can be used to recognize and annotate different segments based on the differences in signal from training examples.

In some other examples, the ultrasound signals can be compared to normative data in addition to, or in alternative to, baseline data. Such normative data may include data associated with expected normal airflow in a healthy patient, such as expected normal airflow velocities in a healthy patient, expected tissue motion measured using B-mode ultrasound, and so on. As one example, this normative data can be provided from a database of measured, normal airflow, which may have been reported in previous clinical or scientific studies. In examples where the ultrasound signals are compared to normative data, the suitable correlation or other algorithm can be used to identify critical changes in the subject's airflow relative to the normative data. By comparing the ultrasound signals to normative data, it is contemplated that additional information about the patient can be provided to the clinician.

For example, if comparing the imaged motion signals to normative data indicates a significant deviation from the normative data, this deviation can be quantified or qualified and presented to the clinician. Such deviations provide information to a clinician that may indicate an underlying respiratory issue, such as an undiagnosed restrictive airway disease, or the like.

As another example, in the emergency room setting, comparing the imaged motion signals to normative data can also provide information to a clinician that may indicate whether the patient is in respiratory distress. This information may help inform a clinician whether to administer emergent sedation in preparation for intubation (e.g., placing an endotracheal breathing tube) to provide respiratory support, or may help a clinician monitor treatment provided to a patient (e.g., whether a patient has an allergic response to a treatment).

It is noted that while ultrasound signals are being acquired, additional physiological data can also be measured. For example, electrocardiograms can be recorded using ECG leads to monitor respiratory impedance and respiratory phases. Although these additional physiological data are not necessary to monitor airflow or to detect airway obstruction or respiratory compromise and/or failure, they can supplement the ultrasound-based data and provide additional information to be relied upon. Additionally or alternatively, other clinical data can also be measured and used to improve the quantitation of airflow. For instance, these clinical data can include body mass index ("BMI"), sleep apnea history, demographics (e.g., age), and physiological measurements (e.g., radius of trachea, ECG signals, as noted above).

As indicated at step 106, B-mode motion signal data are generated from the ultrasound signals. There are numerous ways to perform motion estimation using ultrasound imaging including: color-flow Doppler, normalized cross-correlation, non-rigid affine registration, as well as others. These methods differ in their numerical approach to motion estimation, their computational complexity, and the information which they provide. As one example, the B-mode motion signal data can be generated using non-rigid affine registration, which can be highly correlated to volumetric flow. In some embodiments, the B-mode motion signal data can be analyzed in conjunction with Doppler signal data.

In still other examples, the B-mode motion signal data can be generated using a machine learning algorithm. For instance, the ultrasound signals can be input to a suitable trained machine learning algorithm, such as a suitably trained neural network, generating output as the B-mode motion signal data. In this way, the ultrasound signals are input to the trained machine learning algorithm and the output B-mode motion signal data indicate or otherwise depict the motion based on displacement occurring within the selected ROI.

Thus, the B-mode motion signal data are compared to baseline signal data, as indicated at step 108 to generate respiratory parameter data. Additionally or alternatively, the respiratory parameter data may be computed directly from the B-mode motion data. For example, respiratory rate and other respiratory physiology parameters can be computed directly from the B-mode motion signal data.

In those instances where the B-mode motion signal data are compared with baseline signal data, the baseline signal data can be provided to the ultrasound system or to a computer system in communication with the ultrasound system for this comparison, as indicated at step 110. For example, the baseline signal data can be ultrasound signals acquired from the patient before the patient undergoes a medical procedure. That is, the baseline signal data can be acquired before the patient is administered an anesthetic agent. In some instances, the baseline signal data can include model, or normative, data corresponding to expected normal respiration for a particular patient population group, which may include ultrasound signals acquired from a different patient or subject (e.g., an age-matched, gender-matched, and/or BMI-matched patient). In other examples, the baseline signal data can include previously acquired portions of the ultrasound signals acquired in step 104. For instance, in a real-time monitoring application the most currently acquired ultrasound signals can be compared to ultrasound signals acquired in previous time points. As one example, a sliding window analysis could be performed, in which ultrasound signals acquired within a current time window are compared with ultrasound signals acquired outside of (i.e., prior to) the time window. In these instances, cumulative changes, or a series of changes, in the parameters of the ultrasound signals can be monitored, such that a trending change in the parameters can be identified.

As will be described below, comparing the ultrasound signals with the baseline signal data can generally result in generating an output that indicates a characteristic of airflow in the subject's airway, as indicated at step 112. The characteristic of airflow in the subject's airway may include or otherwise indicate airflow changes, air pressure changes, sound pressure changes, or combinations thereof. For example, the characteristic of airflow may include velocity; airflow pattern (e.g., turbulent versus laminar flow); a quantification of turbulence; acceleration of flow; deceleration of flow; air pressure fluctuations; sound pressure gradient changes at an air-media interface; or combinations thereof.

As other examples, the characteristic of airflow in the subject's airway may include quantitative estimates of airflow, respiratory rate, tidal volume, minute ventilation (i.e., the product of respiratory rate and tidal volume), flow properties such as velocities and Reynolds numbers, respiratory effort (e.g., with how much vigor the patient is breathing), and other such parameters.

As still other examples, the characteristic of airflow in the subject's airway may include an identification of whether the ultrasound signals were acquired during an inspiratory phase or expiratory phase of breath. For instance, determining which phase a flow change happens in can be of diagnostic value. As one example, asthma is an expiratory problem, while sleep apnea or croup are inspiratory problems. The directionality of the B-mode motion can facilitate determining in which phase the pathology may lay and can be of diagnostic value.

As still other examples, the characteristic of airflow in the subject's airway may include an identification of apnea (e.g., no flow at all), central apnea (e.g., when centrally/brain-regulated breath effort is completely stopped, meaning no flow and no B-mode motion), obstructive apnea (e.g., when centrally/brain-regulated breath effort is still intact, and the body is trying to breath against a closed upper airway structure and hence is showing airway motion, meaning no flow and degree of B-mode motion), and/or hypopnea (e.g., when there is less than normal breathing, B-mode motion will show less motion, such as via lesser distance of pixel movement).

In some instances, outputting the characteristic of airflow in the subject's airway may include generating the output as an alarm to the user, such as when a threshold change is detected. In other instances, this includes generating the output as an indication of a quantification or characterization of the airflow. As one example, the output may include images or other visual indications that are displayed to a user, such as via graphical user interface. Such images or visual indications can be overlaid with or displayed alongside images generated from the ultrasound signals, or with other images obtained with the ultrasound system (e.g., B-mode images).

As another example, the ultrasound signals can be correlated with the baseline signal data, and portions of the ultrasound signals that correlate with the baseline signal data can be compared. As above, when changes in the correlated portions of the ultrasound signals and the baseline signal data exceed a threshold, an alarm can be provided to a user.

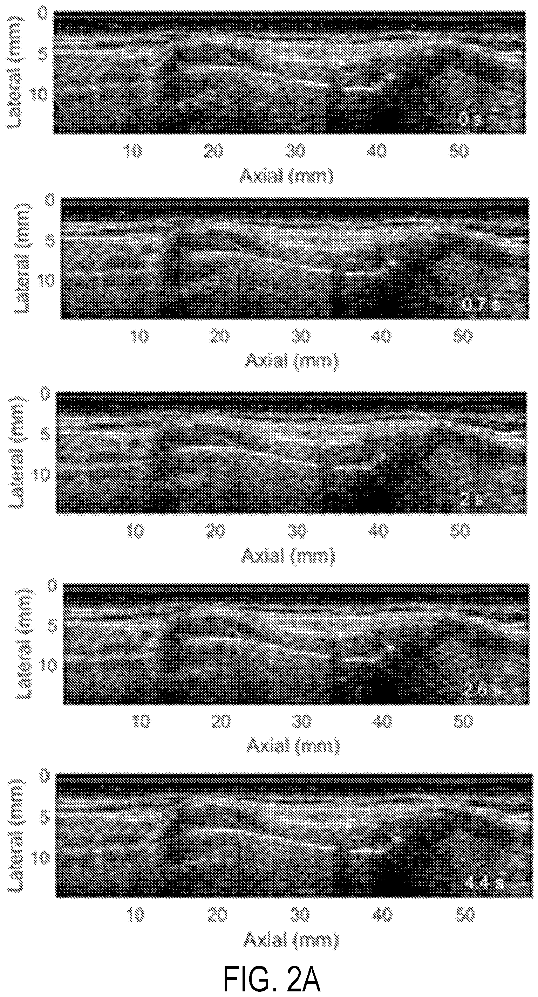

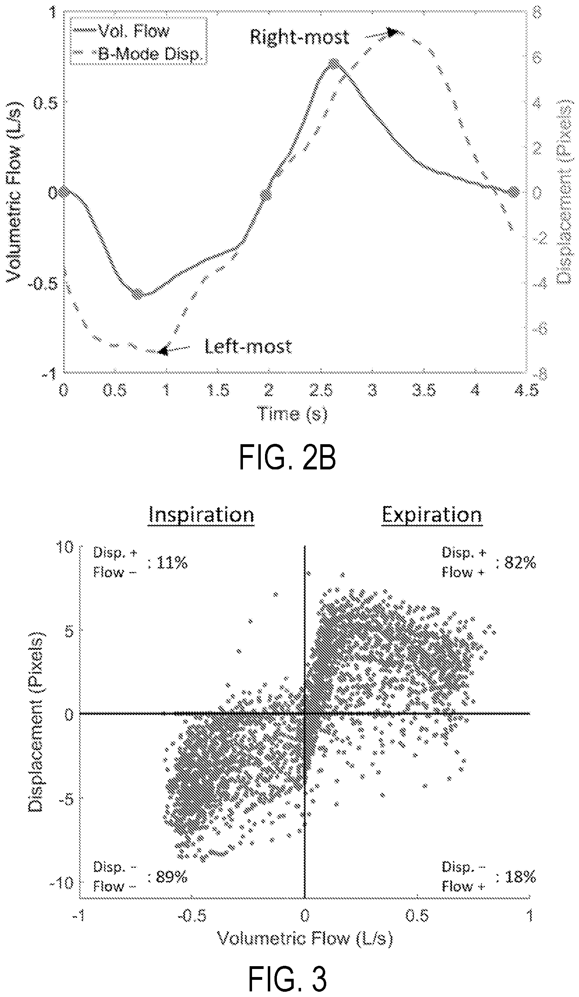

Referring now to FIG. 2A, a series of B-mode images taken during respiration in an example study is shown. There is a small motion of the respiratory tract as the subject breathes through their respiration cycle. Analysis of the displacement in the B-mode image provides additional information regarding the phases or respiration. An example of displacement measured from the B-mode images (e.g., B-mode motion data) is shown in FIG. 2B. In FIG. 2B, the circles on the volumetric flow chart correspond to the individual time-points of the B-mode images (time is also shown in the bottom right corner of the B-mode images in FIG. 2A). Displacement is estimated by the relative motion of a frame 2.5 seconds before. Inspiratory phases can be estimated by looking at whether the estimated displacement is to the left (negative) or the right (positive).

As an example, FIG. 3 illustrates B-mode displacement as a function of volumetric flow for a participant in an example study. In particular, FIG. 3 shows a scatter plot of B-mode displacement as a function of volumetric flow while using an ultrasound system to image the cricothyroid membrane. Each dot represents the displacement and flow during a 0.073 second time-segment; the total amount of data shown in FIG. 3 is for a five minute examination. Percentages indicate the percentage of time when the sign of the displacement matched the phase of respiration (the sign of the flow). Inspiration is defined as negative volumetric flow while expiration is defined as positive volumetric flow.

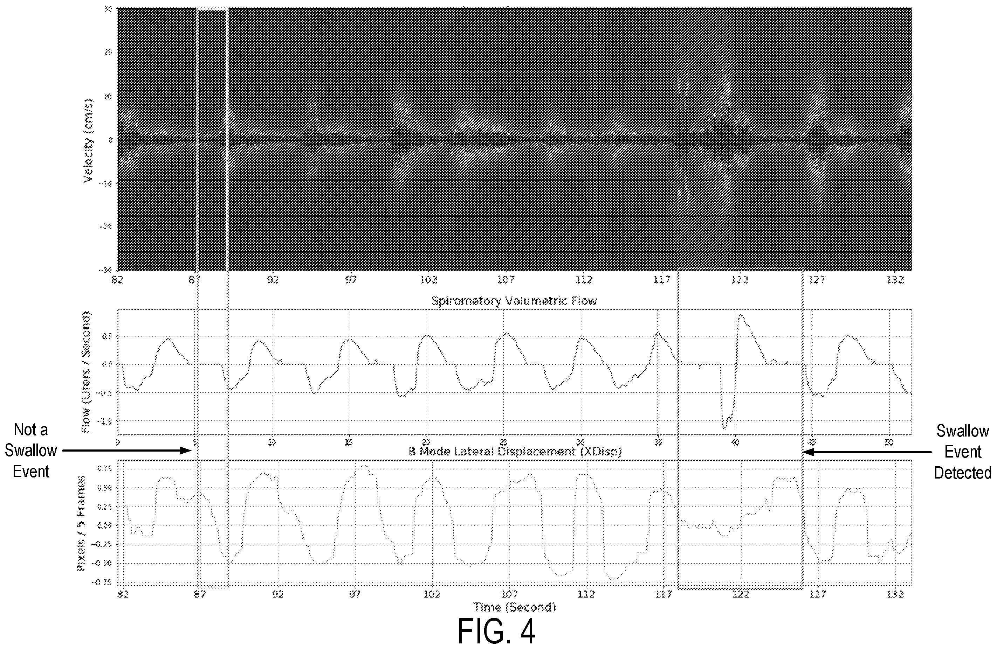

In some implementations, the B-mode motion signal data can be analyzed to determine the presence (e.g., initiation, duration, and termination) of a swallow event. For instance, a swallow event can be detected based on strong signal and noise with zero flow. The swallow signals can then in some instances be removed from the otherwise regular breathing signals before analyzing the B-mode motion signals to quantify or otherwise characterize respiratory parameter data. As shown in FIG. 4, during swallow there is a very violent and very quick motion of the B-mode picture while the flow decreases to zero. This is different in apnea events where this very quick motion is not present.

In some instances, comparing the ultrasound signals to baseline signal data at step 108 can include inputting the ultrasound signals or B-mode motion signal data to a deep learning model, or other suitable machine learning algorithm, that was trained with baseline signal data. For example, a deep learning model, supervised machine learning algorithm, or other suitable machine learning algorithm, can be trained on baseline signal data to predict, classify, or otherwise detect different breathing patterns based on acquired ultrasound signals. The machine learning algorithms can also be trained to compute flow parameters from the ultrasound signals.

The trained machine learning algorithm can include or otherwise implement a long short-term memory ("LSTM") model, a support vector machine ("SVM"), a random forest, a conditional random field, a hidden Markov model, a neural network, and other deep learning models or algorithms.

In some instances, a machine learning algorithm can be trained on baseline signal data that has been annotated or otherwise labeled. For example, baseline signal data can be annotated or otherwise labeled to identify different breathing patterns, including normal, fast, shallow, deep, slow, Valsalva, and so on.

Comparing the B-mode motion signal data to the baseline signal data in step 108 may also include generating an output that characterizes the airflow in the subject's airway. For instance, comparing the ultrasound signals to baseline signal data may identify airflow as turbulent or laminar airflow. This information can be reported to the clinician, such as by generating a display that may form a part of a graphical user interface. As noted, the comparison of the ultrasound signals with the baseline data may include inputting the ultrasound signals to a machine learning algorithm that has been trained on the baseline signal data. In these instances, the machine learning algorithm can generate output that characterizes the airflow in the subject's airway. As one example, the trained machine learning algorithm can generate output as annotated ultrasound signals, which are labeled with a characterization of the airflow being turbulent or laminar.

In addition to characterizing whether airflow is turbulent or laminar, the degree of turbulence can be determined and reported. For instance, comparing the ultrasound signals to the baseline signal data can include generating output that quantifies or otherwise characterizes the degree of turbulent airflow, such as the degree of turbulence at an air-wall interface of the subject's airway. As described above, the comparison of the ultrasound signals with the baseline data may include inputting the ultrasound signals to a machine learning algorithm that has been trained on the baseline signal data. In these instances, the machine learning algorithm can generate output that characterizes or otherwise quantifies the degree of turbulence.

In some other instances, the degree of turbulence can be determined or otherwise characterized using a waveform analysis of the ultrasound signals. For instance, the strength, shape, and pattern of waveforms in the ultrasound signals can be used to quantify or otherwise characterize the degree of turbulence in the ultrasound signals.

In still other instances, other parameters of airflow can be quantified or characterized by comparing the ultrasound signals to baseline signal data, which as noted may include training a suitable machine learning algorithm on the baseline signal data. As one non-limiting example, multivariate linear regression ("MVR") and random forest ("RF") regression can be applied to predict the flow magnitude of inspiration and expiration. Predicting flow values can aid in the detection of early obstruction and can also provide additional data about the information content of the ultrasound signal in respect to flow.

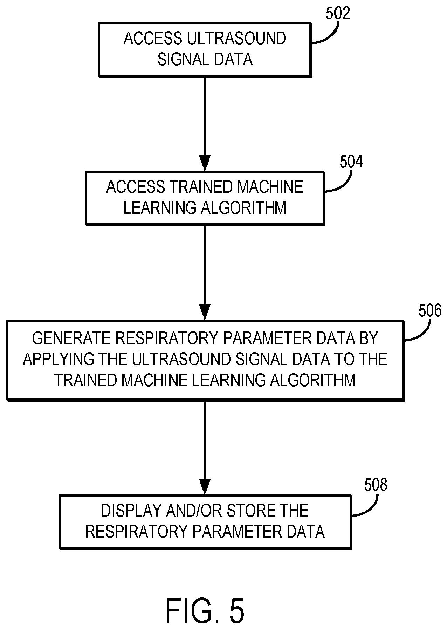

Referring now to FIG. 5, a flowchart is illustrated as setting forth the steps of an example method for measuring one or more parameters associated with a subject's respiration using a suitably trained machine learning algorithm.

The method includes accessing ultrasound signal data (e.g., B-mode signal data) with a computer system, as indicated at step 502. Accessing the ultrasound signal data may include retrieving such data from a memory or other suitable data storage device or medium. Alternatively, accessing the ultrasound signal data may include acquiring such data with an ultrasound system and transferring or otherwise communicating the data to the computer system, which may be a part of the ultrasound system.

A trained machine learning algorithm is then accessed with the computer system, as indicated at step 504. Accessing the trained machine learning algorithm may include accessing model parameters (e.g., weights, biases, or both) that have been optimized or otherwise estimated by training the machine learning algorithm on training data. In some instances, retrieving the machine learning algorithm can also include retrieving, constructing, or otherwise accessing the particular machine learning model (e.g., a neural network architecture) to be implemented. For instance, data pertaining to the layers in the neural network architecture (e.g., number of layers, type of layers, ordering of layers, connections between layers, hyperparameters for layers) may be retrieved, selected, constructed, or otherwise accessed. As one non-limiting example, the machine learning algorithm can be a recurrent neural network, such as an LSTM network.

In general, the machine learning algorithm is trained, or has been trained, on training data in order to quantify or otherwise estimate respiratory parameters or data based on input B-mode signal data.

The ultrasound signal data are then input to the trained machine learning algorithm, generating output as respiratory parameter data, as indicated at step 506. For example, the respiratory parameter data may include estimated values of airflow, respiratory rate, tidal volume, minute ventilation, flow velocity, Reynolds number, respiratory effort, or other such parameters that can quantify one or more aspects of respiratory function. In still other examples, the respiratory parameter data may include an output indicating patterns or information related to the subject's respiration, such as whether the ultrasound signal data correspond to an inspiratory and/or expiratory phase of breath, or such as whether the airflow is laminar or turbulent. In some instances, the output respiratory parameter data may include feature maps associated with the subject's respiration. For instance, the feature maps may depict the spatial distribution or spatial patterns of features, statistics, or other parameters associated with the estimated values described above.

As one example, the feature maps may indicate the local probability for a particular classification (i.e., the probability that a voxel belongs to a particular class), such as laminar or turbulent flow; inspiratory or expiratory phase; or one of apnea, central apnea, obstructive apnea, or hypopnea.

As another example, the feature maps may indicate a classification of the input ultrasound signal data (e.g., by assigning a particular classification to each voxel in the feature map). For instance, the trained neural networks may be trained to implement automatic pattern recognition to generate feature maps that classify laminar or turbulent flow on the ultrasound signal data; or inspiratory or expiratory phase on the ultrasound signal data.

The respiratory parameter data generated by inputting the ultrasound signal data to the trained machine learning algorithm(s) can then be displayed to a user, stored for later use or further processing, or both, as indicated at step 508. In some instances, the output respiratory parameter data can be analyzed to determine whether an alarm (e.g., a visual alarm, an auditory alarm, or both) should be presented to a user.

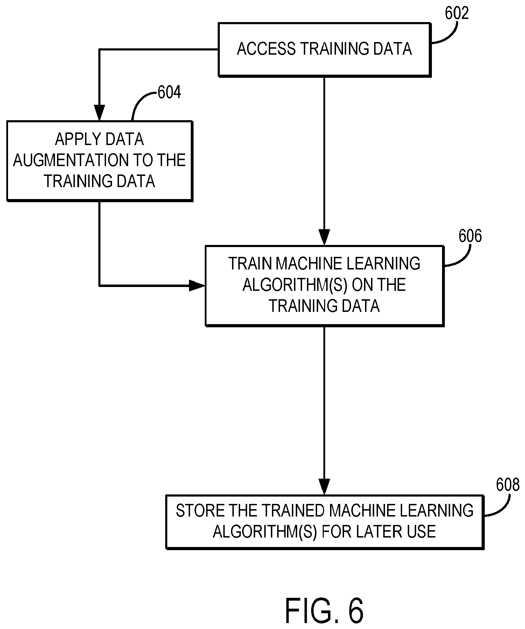

Referring now to FIG. 6, a flowchart is illustrated as setting forth the steps of an example method for training one or more machine learning algorithms on training data, such that the one or more neural networks are trained to receive input as ultrasound signal data in order to generate output as displacement data, tissue motion data, and/or respiratory parameter data.

The machine learning algorithm being trained may include neural network(s) that can implement any number of different neural network architectures (e.g., a convolutional neural network, a residual neural network, a recurrent neural network); a deep learning model; a supervised learning model, an unsupervised learning model, and so on. As one additional example, the machine learning algorithm may implement or be based on a random forest model. As another non-limiting example, the machine learning algorithm may implement an LSTM network.

The method includes accessing training data with a computer system, as indicated at step 602. Accessing the training data may include retrieving such data from a memory or other suitable data storage device or medium. Alternatively, accessing the training data may include acquiring such data with an ultrasound system and transferring or otherwise communicating the data to the computer system, which may be a part of the ultrasound system.

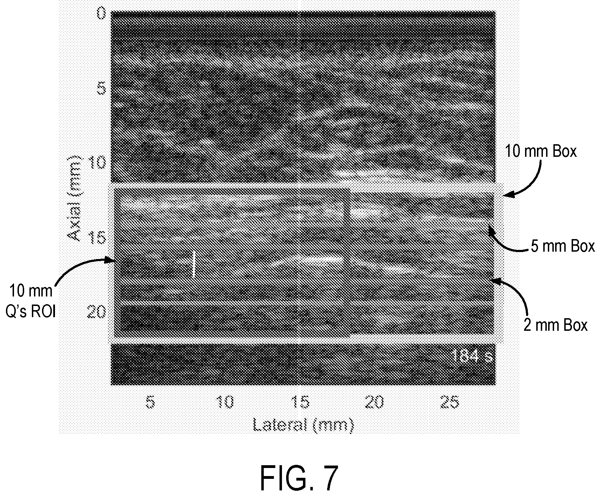

As one example, the training data can include B-mode image data and/or B-mode motion data (e.g., B-mode displacement data) acquired from one or more subjects using various different B-motion window sizes. Additionally or alternatively, the training data can include volumetric flow velocity data, which may also be acquired from the same subject(s) using various different B-motion window sizes. An example of different B-window sizes is shown in FIG. 7. In still other instances, the training data may additionally include clinical data (e.g., BMI, sleep apnea history), demographic data (e.g., age), and/or physiological data (e.g., radius of trachea, ECG) to improve quantitation of air flow.

Additionally or alternatively, the method can include assembling training data into an appropriate data structure on which the machine learning algorithm can be trained. For instance, assembling the training data may include generating labeled data and including the labeled data in the training data. Labeled data may include ultrasound signal data or other relevant data that have been labeled as belonging to, or otherwise being associated with, one or more different classifications or categories. For instance, labeled data may include ultrasound signal data and/or segmented ultrasound signal data that have been labeled based on one or more respiratory parameters or classifications. The labeled data may include data that are classified on a voxel-by-voxel basis, or a regional or larger volume basis.

In some instances, the training data may be augmented before training the machine learning algorithm. In these instances, as part of the data augmentation process, cloned data can be generated from the training data, as indicated at step 604. As an example, the cloned data can be generated by making copies of the training data while altering or modifying each copy of the training data. For instance, cloned data can be generated using data augmentation techniques, such as adding noise to the original training data, performing a deformable transformation (e.g., translation, rotation, both) on the original training data, smoothing the original training data, applying a random geometric perturbation to the original training data, filtering the original training data, combinations thereof, and so on.

One or more machine learning algorithms are then trained on the training data, as indicated at step 606. As one example, the machine learning algorithm can be trained by optimizing model parameters (e.g., weights, biases, or both) based on minimizing a loss function. As one non-limiting example, the loss function may be a mean squared error loss function.

Training a machine learning algorithm may include initializing the machine learning algorithm, such as by computing, estimating, or otherwise selecting initial model parameters (e.g., weights, biases, or both). Training data can then be input to the initialized machine learning algorithm, generating output as respiratory parameter data. The quality of the respiratory parameter data can then be evaluated, such as by passing the respiratory parameter to the loss function to compute an error. The current machine learning algorithm can then be updated based on the calculated error (e.g., using backpropagation methods based on the calculated error). For instance, the current machine learning algorithm can be updated by updating the model parameters (e.g., weights, biases, or both) in order to minimize the loss according to the loss function. When the error has been minimized (e.g., by determining whether an error threshold or other stopping criterion has been satisfied), the current machine learning algorithm and its associated model parameters represent the trained machine learning algorithm.

The one or more trained machine learning algorithm are then stored for later use, as indicated at step 608. Storing the machine learning algorithm(s) may include storing model parameters (e.g., weights, biases, or both), which have been computed or otherwise estimated by training the machine learning algorithm(s) on the training data. Storing the trained machine learning algorithm(s) may also include storing the particular model structure (e.g., neural network architecture) to be implemented. For instance, data pertaining to the layers in the neural network architecture (e.g., number of layers, type of layers, ordering of layers, connections between layers, hyperparameters for layers) may be stored.

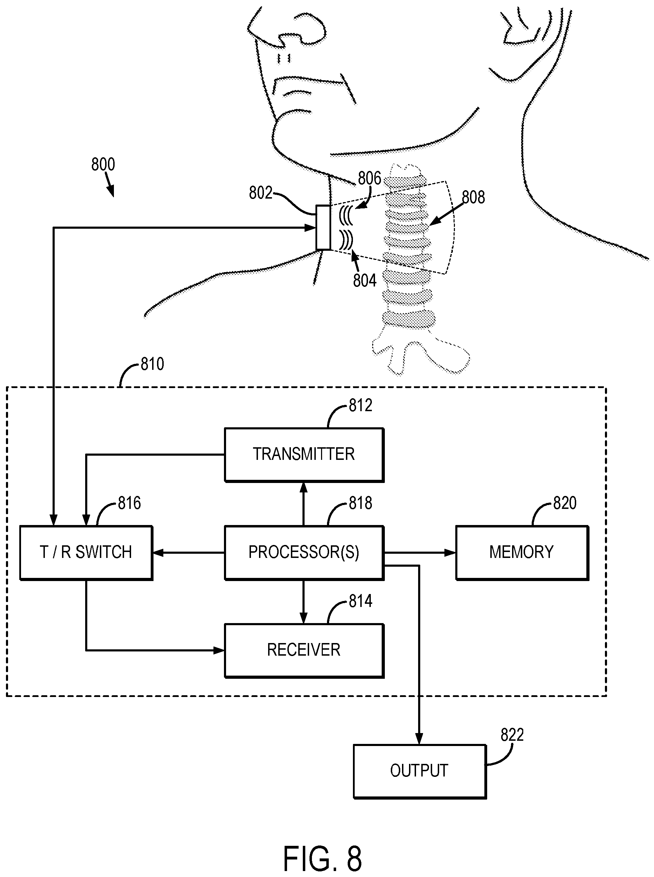

FIG. 8 illustrates the main components of an example airway monitor 800 that can implement the methods described here. In general, the airway monitor 800 can implement an ultrasound system that includes an ultrasound transducer 802 that transmits ultrasonic waves 804 and receives ultrasonic echoes (e.g., ultrasound waves 806) from an anatomical region 808 of a patient, which may be a tracheal wall, a cricothyroid ligament, or other suitable connective or cartilaginous tissue in the trachea, larynx, or pharynx. The ultrasound transducer 802 is generally controlled by a controller 810.

The ultrasound transducer 802 can include a plurality of separately driven transducer elements, and can include any suitable ultrasound transducer array, including linear arrays, curved arrays, phased arrays, and so on. Similarly, the ultrasound transducer 802 can include a 1D transducer, a 1.5D transducer, a 1.75D transducer, a 2D transducer, a 3D transducer, and so on.

When energized by a transmitter 812, the ultrasound transducer 802 produces a burst of ultrasonic energy (e.g., ultrasonic waves 804). The ultrasonic energy reflected back to the ultrasound transducer 802 (e.g., an echo, or ultrasonic waves 806) from the anatomical region 808 is converted to an electrical signal (e.g., an echo signal) by the ultrasound transducer 802 and can be applied separately to a receiver 814 through a set of switches 816. The transmitter 812, receiver 814, and switches 816 are operated under the control of one or more processors 818. The transmitter 812, receiver 814, and switches 816 can be collectively referred to as an acquisition system.

In some instances, the ultrasound transducer 802 can be configured to be positioned or otherwise arranged on the subject's neck, allow for hands-free operation of the airway monitor 800 during a procedure. As one example, the ultrasound transducer 802 can be made to be coupled to the subject's skin surface, such as using an adhesive, a strap, or the like. As another example, the transducer 802 may be composed of piezoelectric crystals or capacitive micro-machined ultrasonic transducers ("CMUT") that are embedded into a gelatinous media that adheres to the subject's skin. Such a transducer 802 can communicate via a wireless or wired connection.

The transmitter 812 can be programmed to transmit ultrasound waves for B-mode imaging, continuous wave Doppler imaging, pulsed wave Doppler imaging, or combinations thereof. The receiver 814 can be programmed to implement a suitable detection sequence for acquiring B-mode ultrasound signal data.

In some configurations, the transmitter 812 and the receiver 814 can be programmed to implement a high frame rate. For instance, a frame rate associated with an acquisition pulse repetition frequency ("PRF") of at least 100 Hz can be implemented. In some configurations, the airway monitor 800 can sample and store at least one hundred ensembles of echo signals in the temporal direction. The airway monitor 800 can implement a detection sequence that includes one of conventional line-by-line scanning, compounding plane wave imaging, compounding diverging beam imaging, continuous wave Doppler imaging, and pulsed wave Doppler imaging.

A scan can be performed by setting the switches 816 to their transmit position, thereby directing the transmitter 812 to be turned on momentarily to energize the ultrasound transducer 802 to transmit ultrasound waves 804 to the anatomical region 808. The switches 816 can then be set to their receive position and the subsequent echo signals produced by the ultrasound transducer 802 in response to one or more detected echoes (e.g., ultrasound waves 806) are measured and applied to the receiver 814. The separate echo signals from the transducer elements in the ultrasound transducer 802 can be combined in the receiver 814 to produce a single echo signal.

The echo signals (e.g., B-mode echo signal data, Doppler ultrasound signals) are communicated to one or more processors 818 to process B-mode signals, Doppler ultrasound signals, or images generated from such signals. As an example, the one or more processor 818 can be programmed to process the B-mode signals and/or Doppler ultrasound signals to implement the methods described in the present disclosure for generating images that depict the anatomical region 808 of the patient, for measuring parameters of B-mode ultrasound signals recorded from the anatomical region 808 of the patient, and for comparing those parameters with similar parameters from baseline signal data provided to the one or more processors 818. The one or more processors 818 can be in communication with a memory 820 that contains the baseline data described above, and which can store B-mode ultrasound signals acquired by the airway monitor 800 and other suitable data. In some instances, the memory 820 may be a networked location (e.g., cloud-based storage).

The output from the one or more processors 818 can be provided to an output 822, which can include a display, speaker, or both. For instance, the output 822 can include an alarm, which may be a display for generating a visual alarm, or a speaker for generating an auditory alarm. In some examples, images produced from the ultrasound signals by the one or more processor 818 can be displayed on an output 822 that includes a display.

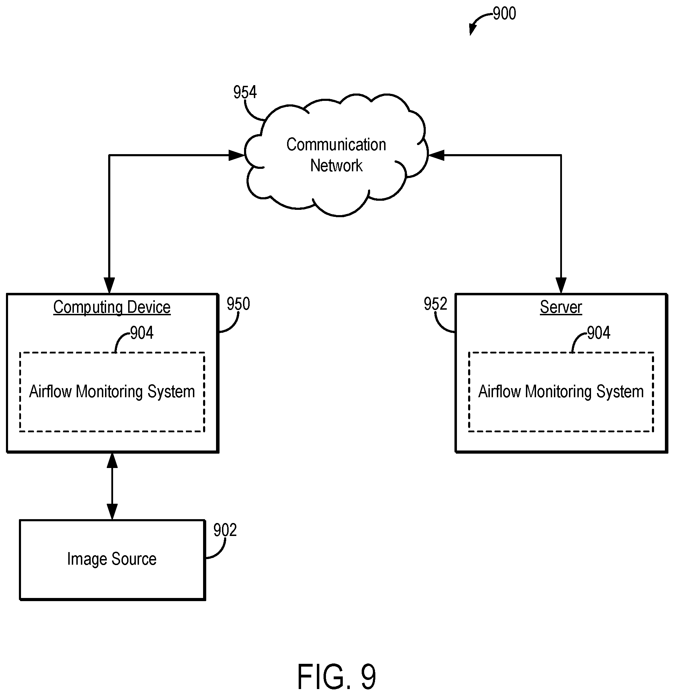

Referring now to FIG. 9, an example of a system 900 for monitoring airflow in a subject in accordance with some embodiments of the systems and methods described in the present disclosure is shown. As shown in FIG. 9, a computing device 950 can receive one or more types of data (e.g., ultrasound signal data) from image source 902, which may be an ultrasound image source. In some embodiments, computing device 950 can execute at least a portion of an airflow monitoring system 904 to monitor airflow in a subject's airway from data received from the image source 902.

Additionally or alternatively, in some embodiments, the computing device 950 can communicate information about data received from the image source 902 to a server 952 over a communication network 954, which can execute at least a portion of the airflow monitoring system 904 to monitor airflow in a subject's airway from data received from the image source 902. In such embodiments, the server 952 can return information to the computing device 950 (and/or any other suitable computing device) indicative of an output of the airflow monitoring system 904 to monitor airflow in a subject's airway from data received from the image source 902.

In some embodiments, computing device 950 and/or server 952 can be any suitable computing device or combination of devices, such as a desktop computer, a laptop computer, a smartphone, a tablet computer, a wearable computer, a server computer, a virtual machine being executed by a physical computing device, and so on. The computing device 950 and/or server 952 can also reconstruct images from the data.

In some embodiments, image source 902 can be any suitable source of image data (e.g., measurement data, images reconstructed from measurement data), such as an ultrasound system, another computing device (e.g., a server storing image data), and so on. In some embodiments, image source 902 can be local to computing device 950. For example, image source 902 can be incorporated with computing device 950 (e.g., computing device 950 can be configured as part of a device for capturing, scanning, and/or storing images). As another example, image source 902 can be connected to computing device 950 by a cable, a direct wireless link, and so on. Additionally or alternatively, in some embodiments, image source 902 can be located locally and/or remotely from computing device 950, and can communicate data to computing device 950 (and/or server 952) via a communication network (e.g., communication network 954).

In some embodiments, communication network 954 can be any suitable communication network or combination of communication networks. For example, communication network 954 can include a Wi-Fi network (which can include one or more wireless routers, one or more switches, etc.), a peer-to-peer network (e.g., a Bluetooth network), a cellular network (e.g., a 3G network, a 4G network, etc., complying with any suitable standard, such as CDMA, GSM, LTE, LTE Advanced, WiMAX, etc.), a satellite communications network (e.g., SATCOM), a radio communications network, a wired network, and so on. In some embodiments, communication network 954 can be a local area network, a wide area network, a public network (e.g., the Internet), a private or semiprivate network (e.g., a corporate or university intranet), any other suitable type of network, or any suitable combination of networks. Communications links shown in FIG. 9 can each be any suitable communications link or combination of communications links, such as wired links, fiber optic links, Wi-Fi links, Bluetooth links, cellular links, and so on.

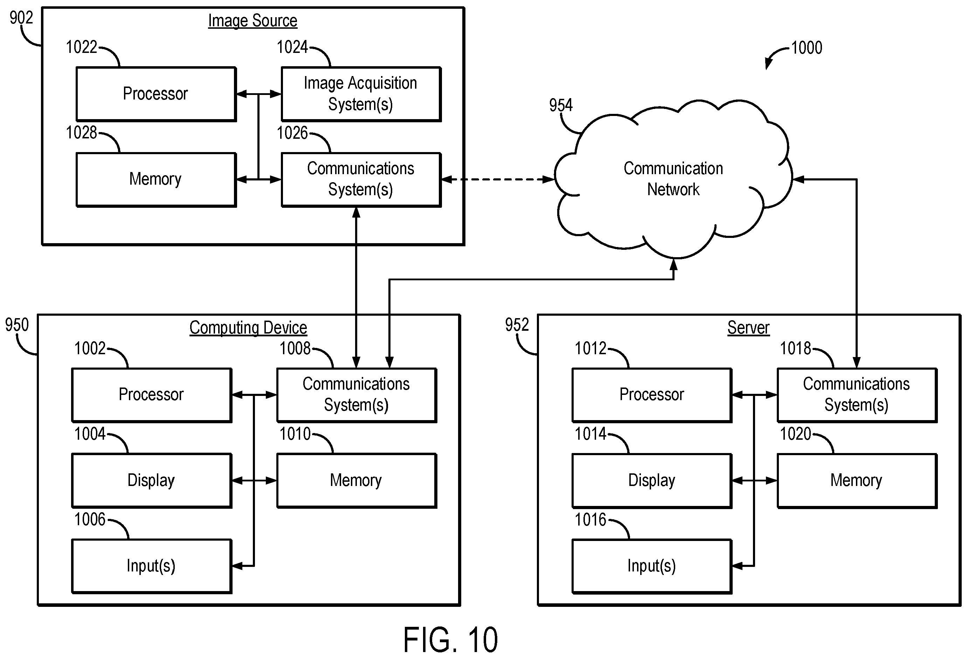

Referring now to FIG. 10, an example of hardware 1000 that can be used to implement image source 902, computing device 950, and server 952 in accordance with some embodiments of the systems and methods described in the present disclosure is shown. As shown in FIG. 10, in some embodiments, computing device 950 can include a processor 1002, a display 1004, one or more inputs 1006, one or more communication systems 1008, and/or memory 1010. In some embodiments, processor 1002 can be any suitable hardware processor or combination of processors, such as a central processing unit ("CPU"), a graphics processing unit ("GPU"), and so on. In some embodiments, display 1004 can include any suitable display devices, such as a computer monitor, a touchscreen, a television, and so on. In some embodiments, inputs 1006 can include any suitable input devices and/or sensors that can be used to receive user input, such as a keyboard, a mouse, a touchscreen, a microphone, and so on.

In some embodiments, communications systems 1008 can include any suitable hardware, firmware, and/or software for communicating information over communication network 954 and/or any other suitable communication networks. For example, communications systems 1008 can include one or more transceivers, one or more communication chips and/or chip sets, and so on. In a more particular example, communications systems 1008 can include hardware, firmware and/or software that can be used to establish a Wi-Fi connection, a Bluetooth connection, a cellular connection, an Ethernet connection, and so on.

In some embodiments, memory 1010 can include any suitable storage device or devices that can be used to store instructions, values, data, or the like, that can be used, for example, by processor 1002 to present content using display 1004, to communicate with server 952 via communications system(s) 1008, and so on. Memory 1010 can include any suitable volatile memory, non-volatile memory, storage, or any suitable combination thereof. For example, memory 1010 can include RAM, ROM, EEPROM, one or more flash drives, one or more hard disks, one or more solid state drives, one or more optical drives, and so on. In some embodiments, memory 1010 can have encoded thereon, or otherwise stored therein, a computer program for controlling operation of computing device 950. In such embodiments, processor 1002 can execute at least a portion of the computer program to present content (e.g., images, user interfaces, graphics, tables), receive content from server 952, transmit information to server 952, and so on.

In some embodiments, server 952 can include a processor 1012, a display 1014, one or more inputs 1016, one or more communications systems 1018, and/or memory 1020. In some embodiments, processor 1012 can be any suitable hardware processor or combination of processors, such as a CPU, a GPU, and so on. In some embodiments, display 1014 can include any suitable display devices, such as a computer monitor, a touchscreen, a television, and so on. In some embodiments, inputs 1016 can include any suitable input devices and/or sensors that can be used to receive user input, such as a keyboard, a mouse, a touchscreen, a microphone, and so on.

In some embodiments, communications systems 1018 can include any suitable hardware, firmware, and/or software for communicating information over communication network 954 and/or any other suitable communication networks. For example, communications systems 1018 can include one or more transceivers, one or more communication chips and/or chip sets, and so on. In a more particular example, communications systems 1018 can include hardware, firmware and/or software that can be used to establish a Wi-Fi connection, a Bluetooth connection, a cellular connection, an Ethernet connection, and so on.

In some embodiments, memory 1020 can include any suitable storage device or devices that can be used to store instructions, values, data, or the like, that can be used, for example, by processor 1012 to present content using display 1014, to communicate with one or more computing devices 950, and so on. Memory 1020 can include any suitable volatile memory, non-volatile memory, storage, or any suitable combination thereof. For example, memory 1020 can include RAM, ROM, EEPROM, one or more flash drives, one or more hard disks, one or more solid state drives, one or more optical drives, and so on. In some embodiments, memory 1020 can have encoded thereon a server program for controlling operation of server 952. In such embodiments, processor 1012 can execute at least a portion of the server program to transmit information and/or content (e.g., data, images, a user interface) to one or more computing devices 950, receive information and/or content from one or more computing devices 950, receive instructions from one or more devices (e.g., a personal computer, a laptop computer, a tablet computer, a smartphone), and so on.

In some embodiments, image source 902 can include a processor 1022, one or more image acquisition systems 1024, one or more communications systems 1026, and/or memory 1028. In some embodiments, processor 1022 can be any suitable hardware processor or combination of processors, such as a CPU, a GPU, and so on. In some embodiments, the one or more image acquisition systems 1024 are generally configured to acquire data, images, or both, and can include an ultrasound transducer. Additionally or alternatively, in some embodiments, one or more image acquisition systems 1024 can include any suitable hardware, firmware, and/or software for coupling to and/or controlling operations of an ultrasound transducer. In some embodiments, one or more portions of the one or more image acquisition systems 1024 can be removable and/or replaceable.

Note that, although not shown, image source 902 can include any suitable inputs and/or outputs. For example, image source 902 can include input devices and/or sensors that can be used to receive user input, such as a keyboard, a mouse, a touchscreen, a microphone, a trackpad, a trackball, and so on. As another example, image source 902 can include any suitable display devices, such as a computer monitor, a touchscreen, a television, etc., one or more speakers, and so on.

In some embodiments, communications systems 1026 can include any suitable hardware, firmware, and/or software for communicating information to computing device 950 (and, in some embodiments, over communication network 954 and/or any other suitable communication networks). For example, communications systems 1026 can include one or more transceivers, one or more communication chips and/or chip sets, and so on. In a more particular example, communications systems 1026 can include hardware, firmware and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, and so on.

In some embodiments, memory 1028 can include any suitable storage device or devices that can be used to store instructions, values, data, or the like, that can be used, for example, by processor 1022 to control the one or more image acquisition systems 1024, and/or receive data from the one or more image acquisition systems 1024; to images from data; present content (e.g., images, a user interface) using a display; communicate with one or more computing devices 950; and so on. Memory 1028 can include any suitable volatile memory, non-volatile memory, storage, or any suitable combination thereof. For example, memory 1028 can include RAM, ROM, EEPROM, one or more flash drives, one or more hard disks, one or more solid state drives, one or more optical drives, and so on. In some embodiments, memory 1028 can have encoded thereon, or otherwise stored therein, a program for controlling operation of image source 902. In such embodiments, processor 1022 can execute at least a portion of the program to generate images, transmit information and/or content (e.g., data, images) to one or more computing devices 950, receive information and/or content from one or more computing devices 950, receive instructions from one or more devices (e.g., a personal computer, a laptop computer, a tablet computer, a smartphone, etc.), and so on.

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (e.g., hard disks, floppy disks), optical media (e.g., compact discs, digital video discs, Blu-ray discs), semiconductor media (e.g., random access memory ("RAM"), flash memory, electrically programmable read only memory ("EPROM"), electrically erasable programmable read only memory ("EEPROM")), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

## Claims

1. A computerized method for analyzing ultrasound signals, the steps of the method comprising:
(a) receiving B-mode signals with a computer system, wherein the B-mode signals were previously acquired using an ultrasound system to acquire the B-mode signals from at least one of connective tissues or cartilaginous tissues in a region-of-interest in a subject's airway;
(b) computing B-mode motion signal data from the B-mode signals using the computer system, wherein the B-mode motion signal data indicate motion that occurred in the region-of-interest while the B-mode signals were acquired; and
(c) estimating from the B-mode motion signal data, respiratory parameter data that characterize airflow in the subject's airway.

2. The method of claim 1, wherein the B-mode motion signal data indicate at least one of displacements occurring in the region-of-interest while the B-mode signals were acquired, or velocities occurring in the region-of-interest while the B-mode signals were acquired.

3. The method of claim 1, wherein the B-mode motion signal data are computed from the B-mode signals using a non-rigid affine registration.

4. The method of claim 1, wherein computing the respiratory parameter data comprises:
accessing a machine learning algorithm that has been trained on training data to generate respiratory parameter data from B-mode motion signal data; and
inputting the B-mode motion signal data to the machine learning algorithm, generating output as the respiratory parameter data.

5. The method as recited in claim 4, wherein the machine learning algorithm is a neural network.

6. The method as recited in claim 5, wherein the neural network is a long short-term memory (LSTM) network.

7. The method as recited in claim 1, wherein the respiratory parameter data indicate a breathing state of the subject, and wherein the breathing state of the subject includes at least one of apnea, central apnea, obstructive apnea, and hypopnea.

8. The method of claim 1, wherein estimating the respiratory parameter data includes comparing the B-mode motion signal data to baseline signal data, generating output as the respiratory parameter data;
wherein comparing the B-mode motion signal data to the baseline signal data comprises comparing a parameter of the B-mode motion signal data with a similar parameter of the baseline signal data; and
further comprising generating an output to a user with the computer system when the parameter of the B-mode motion signal data differs from the similar parameter of the baseline signal data by a selected threshold amount.

9. The method as recited in claim 8, wherein the selected threshold amount is a percent decrease of the parameter relative to the similar parameter.

10. The method as recited in claim 1, wherein estimating the respiratory parameter data includes comparing the B-mode motion signal data to baseline signal data, generating output as the respiratory parameter data;
wherein comparing the B-mode motion signal data to the baseline signal data comprises comparing a parameter of the B-mode motion signal data with a similar parameter of the baseline signal data; and
wherein the parameter is an amplitude of the B-mode motion signal data and the similar parameter is an amplitude of the baseline signal data.

11. The method as recited in claim 1, wherein estimating the respiratory parameter data includes comparing the B-mode motion signal data to baseline signal data, generating output as the respiratory parameter data; and
wherein the baseline signal data is baseline Doppler ultrasound signal data acquired from the subject before acquiring the Doppler ultrasound signal data in step (a).

12. The method as recited in claim 1, wherein the region-of-interest comprises at least one of a cricothyroid ligament, or a tracheal wall.

13. The method of claim 1, further comprising:
receiving Doppler ultrasound signals with the computer system, wherein the Doppler ultrasound signals were previously acquired using the ultrasound system to acquire the Doppler ultrasound signals from the region-of-interest; and
computing the respiratory parameter data using both the B-mode motion signal data and the Doppler ultrasound signals.

14. The method of claim 1, wherein computing the B-mode motion signal data comprises:
accessing a machine learning algorithm that has been trained on training data to generate motion signal data from B-mode signals; and
inputting the B-mode signals to the machine learning algorithm, generating output as the B-mode motion signal data.

## Patentansprüche

1. Computergestütztes Verfahren zur Analyse von Ultraschallsignalen, wobei die Schritte des Verfahrens Folgendes umfassen:
(a) Empfangen von B-Mode-Signalen mit einem Computersystem, wobei die B-Mode-Signale zuvor unter Verwendung eines Ultraschallsystems erfasst wurden, um die B-Mode-Signale von mindestens einem von Bindegewebe oder Knorpelgewebe in einem Untersuchungsbereich in den Atemwegen eines Patienten zu erfassen;
(b) Berechnen von B-Mode-Bewegungssignaldaten aus den B-Mode-Signalen unter Verwendung des Computersystems, wobei die B-Mode-Bewegungssignaldaten eine Bewegung anzeigen, die in dem interessierenden Bereich auftrat, während die B-Mode-Signale erfasst wurden; und
(c) Schätzen von Atemparameterdaten, die den Luftstrom in den Atemwegen des Probanden charakterisieren, aus den B-Mode-Bewegungssignaldaten.

2. Verfahren nach Anspruch 1, wobei die B-Mode-Bewegungssignaldaten mindestens entweder Verschiebungen, die im Untersuchungsbereich während der Erfassung der B-Mode-Signale auftraten, oder Geschwindigkeiten, die im Untersuchungsbereich während der Erfassung der B-Mode-Signale auftraten, angeben.

3. Verfahren nach Anspruch 1, wobei die B-Mode-Bewegungssignaldaten unter Verwendung einer nicht-starren affinen Registrierung aus den B-Mode-Signalen berechnet werden.

4. Verfahren nach Anspruch 1, wobei das Berechnen der Atemparameterdaten umfasst:
Zugreifen auf einen maschinellen Lernalgorithmus, der anhand von Trainingsdaten trainiert wurde, um Atemparameterdaten aus den B-Modus-Bewegungssignaldaten zu generieren; und
Eingeben der B-Mode-Bewegungssignaldaten in den Algorithmus für maschinelles Lernen, Erzeugen einer Ausgabe als die Atemparameterdaten.

5. Verfahren nach Anspruch 4, wobei der Algorithmus für maschinelles Lernen ein neuronales Netzwerk ist.

6. Verfahren nach Anspruch 5, wobei das neuronale Netzwerk ein Long-Short-Term-Memory-Netzwerk (LSTM) ist.

7. Verfahren nach Anspruch 1, wobei die Atemparameterdaten einen Atemzustand der Testperson anzeigen und wobei der Atemzustand der Testperson mindestens eine der folgenden Zustände umfasst: Apnoe, zentrale Apnoe, obstruktive Apnoe und Hypopnoe.

8. Verfahren nach Anspruch 1, wobei das Schätzen der Atemparameterdaten Folgendes umfasst: das Vergleichen der B-Mode-Bewegungssignaldaten mit Basissignaldaten, und das Erzeugen einer Ausgabe als Atemparameterdaten;
wobei das Vergleichen der B-Mode-Bewegungssignaldaten mit den Basissignaldaten das Vergleichen eines Parameters der B-Mode-Bewegungssignaldaten mit einem ähnlichen Parameter der Basissignaldaten umfasst; und
wobei ferner eine Ausgabe an einen Benutzer über das Computersystem erzeugt wird, wenn der Parameter der B-Mode-Bewegungssignaldaten um einen ausgewählten Schwellenwert von dem ähnlichen Parameter der Basissignaldaten abweicht.

9. Das in Anspruch 8 genannte Verfahren, wobei der ausgewählte Schwellenwert eine prozentuale Abnahme des Parameters relativ zu dem ähnlichen Parameter ist.

10. Das Verfahren nach Anspruch 1, wobei das Schätzen der Atemparameterdaten das Vergleichen der B-Mode-Bewegungssignaldaten mit Basis-Signaldaten und das Erzeugen einer Ausgabe als Atemparameterdaten umfasst;
wobei das Vergleichen der B-Mode-Bewegungssignaldaten mit den Basissignaldaten das Vergleichen eines Parameters der B-Mode-Bewegungssignaldaten mit einem entsprechenden Parameter der Basissignaldaten umfasst; und
wobei der Parameter eine Amplitude der B-Mode-Bewegungssignaldaten ist und der ähnliche Parameter eine Amplitude der Basissignaldaten ist.

11. Verfahren nach Anspruch 1, wobei das Schätzen der Atemparameterdaten das Vergleichen der B-Mode-Bewegungssignaldaten mit Basissignaldaten und das Erzeugen einer Ausgabe als Atemparameterdaten umfasst; und
wobei die Basissignaldaten Doppler-Ultraschall-Basissignaldaten sind, die von der Testperson vor der Erfassung der Doppler-Ultraschall-Signaldaten in Schritt (a) erfasst wurden.

12. Verfahren nach Anspruch 1, wobei der Bereich von Interesse mindestens eines von einem Cricothyroid-Ligament oder einer Trachealwand umfasst.

13. Verfahren nach Anspruch 1, das ferner umfasst:
Empfangen von Doppler-Ultraschallsignalen mit dem Computersystem, wobei die Doppler-Ultraschallsignale zuvor unter Verwendung des Ultraschallsystems erfasst wurden, um die Doppler-Ultraschallsignale aus dem Untersuchungsbereich zu erfassen; und
Berechnen der Atemparameterdaten unter Verwendung sowohl der B-Mode-Bewegungssignaldaten als auch der Doppler-Ultraschallsignale.

14. Verfahren nach Anspruch 1, wobei das Berechnen der B-Mode-Bewegungssignaldaten umfasst:
Zugreifen auf einen Algorithmus für maschinelles Lernen, der anhand von Trainingsdaten trainiert wurde, um Bewegungssignaldaten aus B-Mode-Signalen zu generieren; und
Eingabe der B-Mode-Signale in den Algorithmus für maschinelles Lernen, um als Ausgabe die B-Mode-Bewegungssignal-Daten zu generieren.

## Revendications

1. Méthode informatisée pour analyser des signaux ultrasonores, les étapes de la méthode comprenant :
(a) la réception de signaux en mode B avec un système informatique, dans laquelle les signaux en mode B ont été précédemment acquis à l'aide d'un système à ultrasons pour acquérir les signaux en mode B à partir d'au moins l'un parmi des tissus conjonctifs ou des tissus cartilagineux dans une région d'intérêt dans les voies aériennes d'un sujet ;
(b) le calcul de données de signal de mouvement en mode B à partir des signaux en mode B à l'aide du système informatique, dans laquelle les données de signal de mouvement en mode B indiquent un mouvement qui s'est produit dans la région d'intérêt pendant que les signaux en mode B ont été acquis ; et
(c) l'estimation, à partir des données de signal de mouvement en mode B, de données de paramètres respiratoires qui caractérisent le débit d'air dans les voies aériennes du sujet.

2. Méthode selon la revendication 1, dans laquelle les données de signal de mouvement en mode B indiquent au moins l'un parmi des déplacements se produisant dans la région d'intérêt pendant que les signaux en mode B ont été acquis, ou des vitesses se produisant dans la région d'intérêt pendant que les signaux en mode B ont été acquis.

3. Méthode selon la revendication 1, dans laquelle les données de signal de mouvement en mode B sont calculées à partir des signaux en mode B à l'aide d'un enregistrement affine non rigide.

4. Méthode selon la revendication 1, dans laquelle le calcul des données de paramètres respiratoires comprend :
l'accès à un algorithme d'apprentissage automatique qui a été entraîné sur des données d'entraînement pour générer des données de paramètres respiratoires à partir de données de signal de mouvement en mode B ; et
l'entrée des données de signal de mouvement en mode B dans l'algorithme d'apprentissage automatique, générant une sortie en tant que données de paramètres respiratoires.

5. Méthode selon la revendication 4, dans laquelle l'algorithme d'apprentissage automatique est un réseau neuronal.

6. Méthode selon la revendication 5, dans laquelle le réseau neuronal est un réseau de mémoire à long et à court terme (MLCT).

7. Méthode selon la revendication 1, dans laquelle les données de paramètres respiratoires indiquent un état respiratoire du sujet, et dans laquelle l'état respiratoire du sujet inclut au moins l'une parmi l'apnée, l'apnée centrale, l'apnée obstructive et l'hypopnée.

8. Méthode selon la revendication 1, dans laquelle l'estimation des données de paramètres respiratoires inclut la comparaison des données de signal de mouvement en mode B à des données de signal de référence, générant une sortie en tant que données de paramètres respiratoires ;
dans laquelle la comparaison des données de signal de mouvement en mode B aux données de signal de référence comprend la comparaison d'un paramètre des données de signal de mouvement en mode B à un paramètre similaire des données de signal de référence ; et
comprenant en outre la génération d'une sortie vers un utilisateur avec le système informatique lorsque le paramètre des données de signal de mouvement en mode B diffère du paramètre similaire des données de signal de référence d'une quantité seuil sélectionnée.

9. Méthode selon la revendication 8, dans laquelle le seuil sélectionné est une diminution en pourcentage du paramètre par rapport au paramètre similaire.

10. Méthode selon la revendication 1, dans laquelle l'estimation des données de paramètres respiratoires inclut la comparaison des données de signal de mouvement en mode B à des données de signal de référence, générant une sortie en tant que données de paramètres respiratoires ;
dans laquelle la comparaison des données de signal de mouvement en mode B aux données de signal de référence comprend la comparaison d'un paramètre des données de signal de mouvement en mode B à un paramètre similaire des données de signal de référence ; et
dans laquelle le paramètre est une amplitude des données de signal de mouvement en mode B et le paramètre similaire est une amplitude des données de signal de référence.

11. Méthode selon la revendication 1, dans laquelle l'estimation des données de paramètres respiratoires inclut la comparaison des données de signal de mouvement en mode B à des données de signal de référence, générant une sortie en tant que données de paramètres respiratoires ; et
dans laquelle les données de signal de référence sont des données de signal ultrasonore Doppler de référence acquises auprès du sujet avant l'acquisition des données de signal ultrasonore Doppler à l'étape (a).

12. Méthode selon la revendication 1, dans laquelle la région d'intérêt comprend au moins l'un parmi un ligament cricothyroïdien, ou une paroi trachéale.

13. Méthode selon la revendication 1, comprenant en outre :
la réception de signaux ultrasonores Doppler avec le système informatique, dans laquelle les signaux ultrasonores Doppler ont été précédemment acquis à l'aide du système à ultrasons pour acquérir les signaux ultrasonores Doppler à partir de la région d'intérêt ; et
le calcul des données de paramètres respiratoires à l'aide la fois des données de signal de mouvement en mode B et des signaux ultrasonores Doppler.

14. Méthode selon la revendication 1, dans laquelle le calcul des données de signal de mouvement en mode B comprend :
l'accès à un algorithme d'apprentissage automatique qui a été entraîné sur des données d'entraînement pour générer des données de signal de mouvement à partir de signaux en mode B ; et
l'entrée des signaux en mode B dans l'algorithme d'apprentissage automatique, générant une sortie en tant que données de signal de mouvement en mode B.
